# NEW EUROPEAN PATENT SPECIFICATION

(11) **EP 1 493 403 B2**
(45) Date of publication and mention of the opposition decision: **26.03.2008**
(45) Mention of the grant of the patent: 22.02.2006
(21) Application number: 04253360.4
(22) Date of filing: 04.06.2004
(51) Int. Cl.: A61F 2/06, A61M 25/00

(54) **Balloon catheter with self-centering tip**
Ballonkatheter mit selbstzentrierender Spitze
Cathéter à ballonnet avec bout à auto-centrage

(30) Priority: 30.06.2003 US 610053
(43) Date of publication of application: 05.01.2005
(73) Proprietor: Cordis Corporation, Miami Lakes, Florida 33014 (US)
(72) Inventor: Lorenzo, Juan A, Davie, Florida 33328 (US); Marajh, Rajindrah, Plantation, Florida 33317 (US)
(74) Representative: Belcher, Simon James

(56) References cited:
- EP-A- 1 270 040
- WO-A-01/34240
- WO-A-02/26323
- WO-A-03/028794
- US-A- 5 769 819
- US-A- 20 030 032
- US-A1- 2001 014 821
- US-B- 6 190 393
- US-B- 6 206 852
- US-B- 6 403 011
- EuroPCT 2003 Conference Catalogue, pp.3-10,28,29,33,35,45,47

## Description

The present invention relates generally to medical devices, and more particularly to a balloon catheter with a self-centering tip.

Balloon catheters are used in a variety of therapeutic applications, including intravascular catheters for procedures such as angioplasty. Nearly one million angioplasties are performed worldwide each year to treat vascular disease, including coronary, neurological and peripheral blood vessels partially or totally blocked or narrowed by a lesion or stenosis. By way of example, the present invention will be described in relation to coronary and peripheral angioplasty treatments. However, it should be understood that the present invention relates to any balloon catheter having a self-centering tip, and is not limited to angioplasty. Most balloon catheters have a relatively long and flexible tubular shaft defining one or more passages or lumens, and an inflatable balloon attached near one end of the shaft. This end of the catheter where the balloon is located is customarily referred to as the "distal" end, while the other end is called the "proximal" end. The other end of this inflation lumen generally leads to a hub coupling at the proximal end for connecting the inflation lumen to various equipment.

The interior of the balloon is connected to one of the lumen(s) extending through the shaft for the purpose of selectively inflating and deflating the balloon. Structurally, the balloon may define an inflatable central portion defining an inflated size, flanked by a pair of proximal and distal conical portions, flanked by a pair of proximal and distal legs or collars. The proximal and distal collars may be affixed to the shaft.
Examples of this type of balloon catheter are shown in the following patents: United States Patent number 5,304,197, entitled "Balloons For Medical Devices And Fabrication Thereof," issued to Pinchuk et al. on April 19,1994; United States Patent number 5,370,615, entitled "Balloon Catheter For Angioplasty," issued to Johnson on December 6, 1994; and also in United States Patent Application number US 2001/0014821 of Juman et al., entitled "Baloon Catheter And Stent Delivery System Having Enhanced Stent Retantion", published on August 16, 2001, which discloses a balloon catheter stent delivery system as described in the preamble of claim 1.

The catheter shaft also may define a second passage for slidingly receiving a guidewire, referred to as a guidewire lumen. The guidewire lumen extends between a distal guidewire port at the catheter distal end, and a proximal guidewire port. The proximal guidewire port may be located in the proximal hub (referred to as an "over-the-wire" arrangement, or it may be located somewhere between the balloon and the proximal hub (referred to as a "rapid exchange" arrangement).

Common treatment methods for using such a balloon catheter include advancing a guidewire into the body of a patient, by directing the guidewire distal end percutaneously through an incision and along a body passage until it is located within or beyond the desired site. The term "desired site" refers to the location in the patient's body currently selected for treatment by a health care professional. The guidewire may be advanced before, or simultaneously with, a balloon catheter. When the guidewire is within the balloon catheter guidewire lumen, the balloon catheter may be advanced or withdrawn along a path defined by the guidewire. After the balloon is disposed within the desired site, it can be selectively inflated to press outward on the body passage at relatively high pressure to a relatively constant diameter, in the case of an inelastic or non-compliant balloon material.

This outward pressing of a constriction or narrowing at the desired site in a body passage is intended to partially or completely re-open or dilate that body passageway or lumen, increasing its inner diameter or cross-sectional area. In the case of a blood vessel, this procedure is referred to as angioplasty. The objective of this procedure is to increase the inner diameter or cross-sectional area of the vessel passage or lumen through which blood flows, to encourage greater blood flow through the newly expanded vessel. The narrowing of the body passageway lumen is called a lesion or stenosis, and may be formed of hard plaque or viscous thrombus.

For purposes of this description only, the "tip" of a balloon catheter may be defined as that portion extending from the catheter distal end to a point proximal of the balloon distal collar, where the balloon material transitions from being affixed or sealed to the shaft to being free of the shaft, and begins to define the balloon inflatable portion.

This tip may include the length of the balloon distal collar, and also some length of shaft material or separate tip material extending distal of the balloon distal collar. In another possible configuration of the tip, the balloon material in the area of the balloon distal collar extends to the catheter-distal end.

Prior balloon catheters included some tips that were relatively long and flexible, to provide a very flexible tip for more easily following a guidewire path with many bends and branches. The term "relatively long" in this context, for example in a coronary balloon catheter having an inflated size or diameter of about 3.5 millimeters, might include tips having lengths of 4 millimeters or more. To the casual observer, a distal tip length of 4 millimeters may seem small. However, it is worth remembering that for example coronary arteries may range in size (in the different regions of patient anatomy) from 4 millimeters in diameter down to 1 millimeter. In addition, the length of the distal tip may affect one or more of the performance factors listed above and discussed elsewhere.
It is desirable to provide a balloon catheter having an optimum combination of various performance characteristics, which may be selected among: flexibility, lubricity, pushability, trackability, crossability and others. Flexibility may relate to bending stiffness of a medical device (balloon catheter and/or stent, for example) in a particular region or over its entire length, or may relate to the material hardness of the components. Lubricity may refer to reducing friction by using low-friction materials or coatings. Pushability may relate to the column strength of a device or system along a selected path. Trackability may refer to a capability of a device to successfully follow a desired path, for example without prolapse. Crossability may be clarified by understanding that physicians prefer to reach the desired site with the balloon catheter while encountering little or no friction or resistance. Some prior balloon catheters provided an inner tubular body extending through the balloon, a distal seal area where the balloon distal leg is sealed to the inner body, and a tubular tip extending distal of the seal, The tubular tip was formed either by extending the inner body past the seal, or by affixing a separate tubular tip to the seal. Such a design provides a flexible tip that is more flexible than the seal area or the portion of the inner body surrounded by the balloon. Prior designers surmised it desirable to provide such a flexible tip, distal of the less- flexible seal area. The resulting tip assembly would often be at least 4 millimetres or longer.

The distal tip region of a balloon catheter preferably has an optimum balance of various performance characteristics, which may include flexibility, a strong seal to support high balloon inflation pressures, a shape that facilitates easy advancement to the desired site for treatment, and a length selected to be a combination of: (i) flexible to allow bending along a desired path, (ii) long to provide sufficient seal strength, (iii) and short to extend a minimal distance distal of the desired site. It is also desirable to provide a tip assembly that tends to center itself within the body passageway.

These and various other objects, advantages and features of the invention will become apparent from the following description and claims, when considered in conjunction with the appended drawings.

Embodiments of the invention will now be described by way of example with reference to the accompanying drawings, in which:
Figure 1 is a partial external side view of the distal end of a balloon catheter having a stent mounted around the balloon, and a guidewire, arranged according to the principles of the present invention;
Figure 2 is a partial external side view of the distal end of a balloon catheter and stent;
Figure 3 is a partial external side view of the distal end of a balloon catheter and stent;
Figure 4 is a partial external side view of the distal end of a balloon catheter and stent;
Figure 5 is a partial external side view of the distal end of the balloon catheter and stent of Figure 1, showing a curved path;
Figure 6 is a partial external side view of the distal end of the balloon catheter and stent of Figure 2, showing a curved path;
Figure 7 is a partial external side view of the distal end of a balloon catheter;
Figure 8 is an external perspective view of a balloon catheter stent delivery system, and
Figure 9 is a partial external side view of the distal end of a balloon catheter.

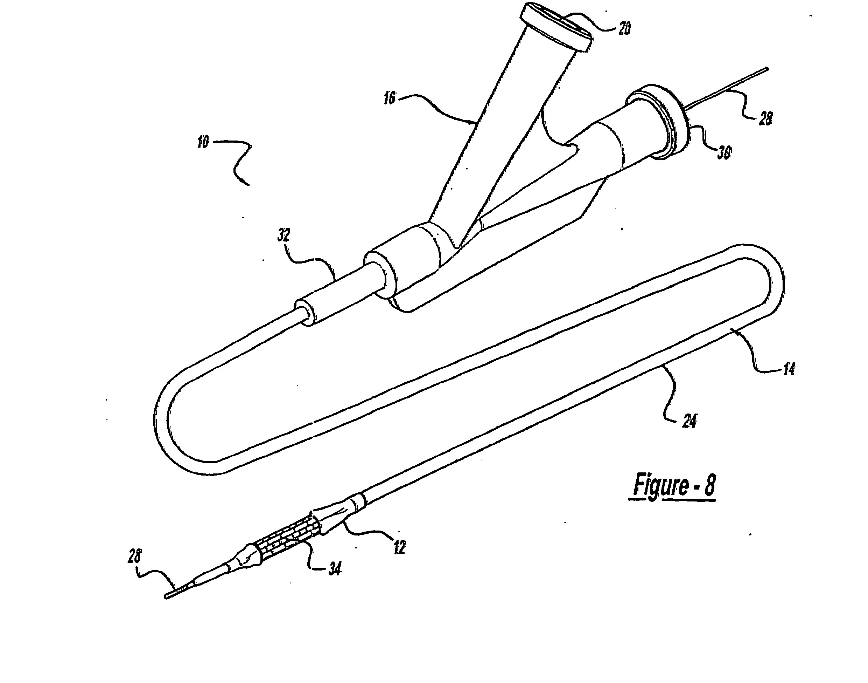

Referring to the drawings, a balloon catheter system is depicted, with one of the preferred embodiments of the present invention being shown generally at 10. The balloon catheter of Figure 8 has an inflatable balloon 12, a relatively long and flexible tubular shaft 14, and a hub 16. The balloon 12 is affixed to the shaft 14 near a distal end of the shaft 14, and the hub 16 is affixed to the proximal end of the shaft 14.

The shaft defines one or more passages or lumens extending through the shaft, at least one of which is an inflation lumen connected to the balloon 12 for the purpose of selectively inflating and deflating the balloon 12. The inflation lumen thus provides fluid communication between the interior of the balloon 12 at the distal end of the inflation lumen, and a hub inflation port 20 having a coupling or luer-look fitting at the proximal end for connecting the inflation lumen to a source of pressurized inflation fluid (not shown) in the conventional manner.

In the illustrated embodiment, the shaft 14 is constructed of an inner and outer tubular body 22 and 24. The inner body 22 defines a guidewire lumen, while the inflation lumen is defined by the annular space between the inner and outer tubular bodies 22 and 24. The guidewire lumen is adapted to receive an elongated flexible guidewire 28 in a sliding fashion, such that the guidewire 28 and catheter 10 may be advanced or withdrawn independently, or the catheter 10 may be guided along a path selected with the guidewire 28. The shaft may of course have various configurations instead of this coaxial design, including a single extruded tube defining any suitable number of parallel side-by-side lumens, a proximal shaft portion formed of a metal hypotube, and others.
The proximal hub 16 is affixed to the proximal end of the shaft 14, and provides an inflation port 20 and a guidewire port 30, again with a luer-lock fitting or hemostatic valve (not shown). Such a valve allows the guidewire 28 to traverse and slide within the guidewire lumen, yet while resisting a loss of blood or other fluids through the guidewire lumen and guidewire port. The inner and outer tubular bodies 22 and 24 are securely received within the hub 16, and are surrounded by a tubular strain relief 32. The hub 16 provides fluid communication between the guidewire lumen and the guidewire port 30, as well as between the annular inflation lumen and the inflation coupling 20.

The balloon 12 in its fully inflated profile shape preferably has a cylindrical working portion with an inflated diameter located between a pair of conical end portions, and a pair of proximal and distal legs or collars affixed to the shaft. In its deflated profile shape, the balloon 12 preferably has several pleats that are wrapped around the shaft. The balloon material is preferably substantially inelastic, and stretches only a relatively small amount under pressures of about 15 atmospheres or more.

Various different materials and techniques are known in the art for making the components of the catheter, including the materials synthetic polyamide such as Nylon, PEEK, thermoplastic elastomer such as Pebax, PET, or a block copolymer thereof. The components may be made by extrusion or co-extrusion, injection molding or vacuum forming for example. The components may be made of a single material or multiple layers, with or without reinforcement.

According to the principles of the present invention, the balloon catheter has an improved self-centering tip. For purposes of this portion of the description only, the term "distal tip" may refer to that portion of the catheter extending distally of a point where the distal leg or collar of the balloon meets the catheter shaft. In other words, the "tip" of a balloon catheter may be described as that portion extending from the catheter distal end to a point proximal of the balloon distal collar, where the balloon material transitions from being affixed to the shaft to being free of the shaft, and begins to define the balloon inflatable portion.
The distal tip of the present invention has a novel design with specific features and a specific shape, which tends to cause the tip to be self-centering as the catheter is advanced to the desired site for treatment. The present invention also tends to inhibit or reduce friction or tactile feedback as the distal portion of the catheter encounters anatomy or a stenosis or lesion, for example.

One of these features is that the distal tip is unusually short, having a length along the longitudinal axis of 2.5 millimetres or less. In addition, the distal tip defines at least two tapering cam surfaces. The first cam surface defines an angle of 5-20 degrees with respect to the longitudinal axis, and is located within 0.254 millimetres of the catheter distal end. The second cam surface defines an angle of 18-35 degrees with respect to the longitudinal axis, and is located within 2.0 millimetres of the catheter distal end. A result of this novel design is that the distal tip tends to self-center itself within the body passageway.

Of various distal tip designs within the scope of the present invention, one embodiment provides a first cam surface that extends proximally from the catheter distal end a distance of at most 0.3 millimeters. Another embodiment, which may or may not accompany this feature, provides a second cam surface that extends a distance from the catheter distal end between 1.5-2.5 millimeters.

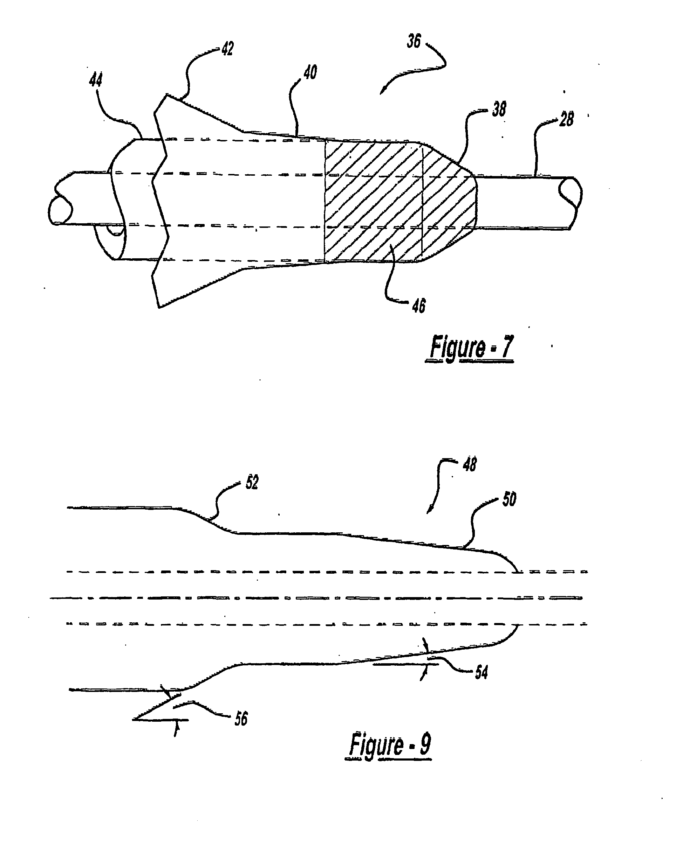

Two embodiments of the present invention are shown in Figures 7 and 9, which are greatly magnified views. The distal tip 36 shown in figure 7 defines a first and second cam surface 38 and 40. The cross-hatching indicates that the materials of balloon 42 and shaft member 44 are melted together, indicated at 46. In other words, the distal seal area is formed of balloon material and shaft material melted together, and forms the distal end of the catheter. The first and second cam surfaces of the present invention are shown.
Figure 9 shows a distal tip 48 in a somewhat diagrammatic cross-section format. Distal tip 48 likewise defines a first and second ramped cam surface 50 and 52, which in turn define angles 54 and 56. Preferably, angle 54 is between 5-20 degrees inclusive, and angle 56 is between 18-35 degrees inclusive.

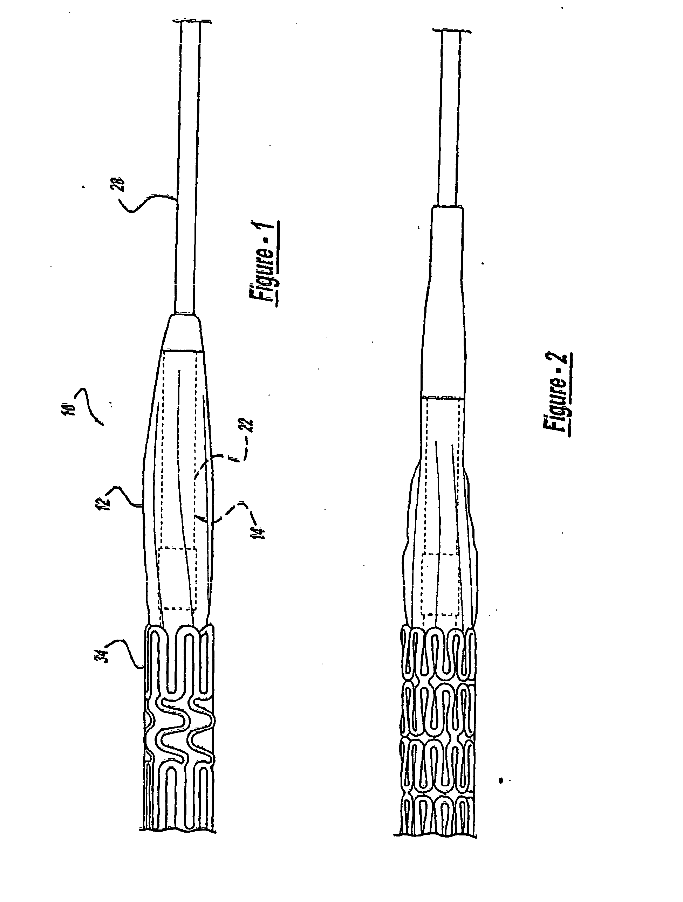

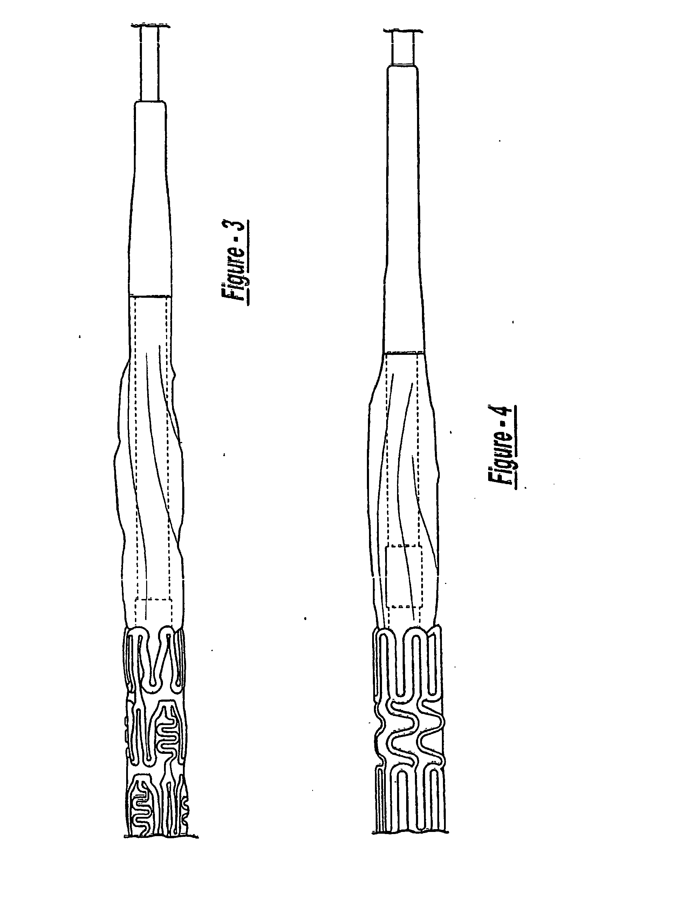

For example, prior balloon catheters may have relatively long distal tips, on the order of about 4 millimeters or more. These relatively long distal tips were also selected with relatively small outer diameters, thin tubular sidewalls, and defined little or no tapering angle with respect to the longitudinal axis, as shown in Figures 2-4. This type of long and thin distal tip may tend to provide a distal edge surface that may catch or provide tactile feedback when it is advanced to the desired site for treatment, which may be a calcified lesion, for example.

However, the novel arrangement of the present invention provides tapering or ramped cam surfaces defining a sufficient angle and having a sufficiently small length, such that the first surface which tends to contact any lesion or stenosis is angled or ramped, and is not a relatively abrupt edge type of surface. As a result, the distal tip of the present invention tends to center itself, whether it is advanced to the desired site alone or over a guidewire. Preferably, the distal tip of the present invention may be constructed to be self-centering when the catheter is advanced along a straight or complicated path. For example, the path to the desired site for treatment may be some combination of straight portions and curved, bending, tortuous or branching portions. In such situations, the cam surfaces tend to present a tapering or ramped surface to first contact any portion of the anatomy.

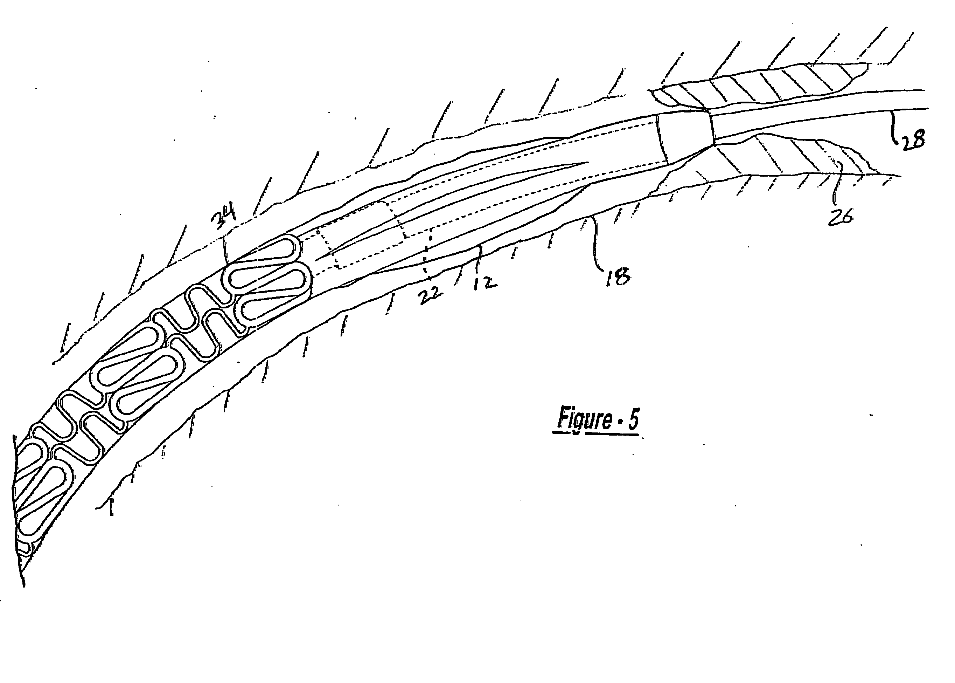

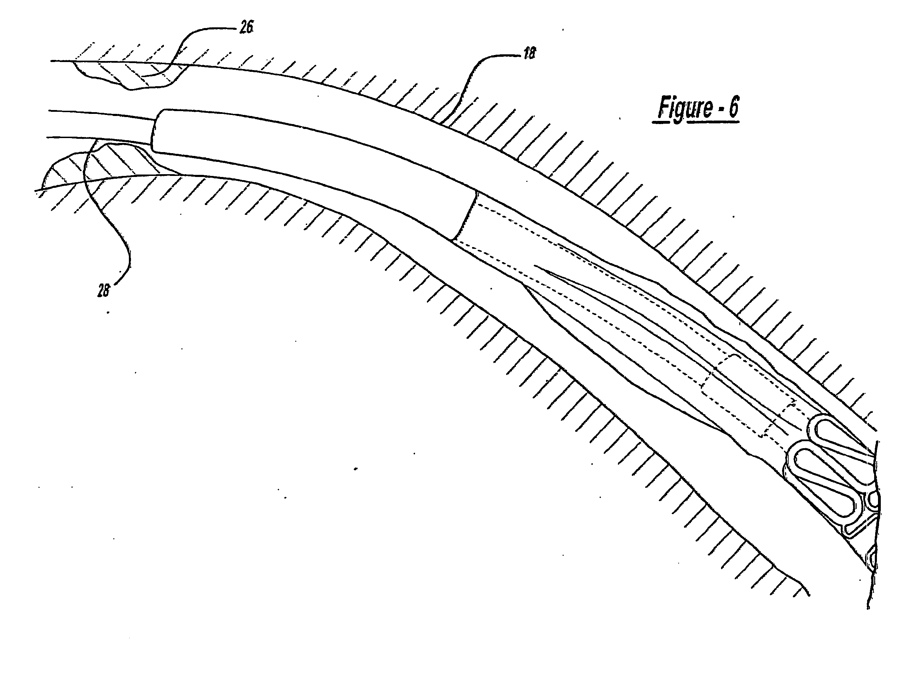

An example of a curved path is shown in Figure 5, in which the tapering distal tip contacts the anatomy in an advantageous way. The ramped surface of the distal tip tends to push the guidewire 28 away from vessel wall 18 in the region of stenosis 26. In contrast, Figure 6 shows a possible arrangement in which a distal tip is not arranged according to the present invention, which has a relatively long distal tip, and provides a leading edge that may catch or cause tactile feedback. In the example of Figure 6, guidewire 28 tends to "apex" a turn, following a path of least resistance and tending to smooth out turns. As illustrated in Figure 6, a distal tip without the characteristics of the present invention may tend to catch or provide tactile feedback when it contacts a stenosis or lesion 26.

The balloon catheter and stent delivery system of the present invention may be made using various methods, many of which are known in the art, including extruding polymer tubes, injection-molding the proximal hub, and extruding a balloon parison and then blowing the parison into a balloon having the desired properties. It is also known to affix polymer components to each other by heat-sealing, or by using an adhesive such as a UV-cured adhesive. The distal tip of the present invention may be formed using new methods, including heat-shaping to simultaneously heat-seal the balloon distal collar to the inner body shaft tube while at the same time shaping the distal tip, for example by using an RF heating die having a tapered inner surface.

Several features of this preferred method of making the balloon catheter stent delivery system of the present invention have Some importance to the performance of the resulting product, including the temperatures, pressures, time periods, crimped outer diameter of the stent, the internal diameter of the mold, as well as the thermal characteristics of the balloon, stent and mold.

A stent of any suitable type or configuration may be provided with the catheter of the present invention, such as the well-known Palmaz-Schatz balloon expandable stent and the successful stent sold under the mark BX velocity. Various kinds and types of stents are available in the market, and many different currently available stents are acceptable for use in the present invention, as well as new stents which may be developed in the future. The stent 34 depicted in the drawings is a cylindrical metal mesh stent having an initial crimped outer diameter, which may be forcibly expanded by the balloon 12 to a deployed diameter. When deployed in a body passageway of a patient, the stent 34 may be designed to preferably press radially outward to hold the passageway open.

It should be understood that an unlimited number of configurations for the present invention could be realized. The foregoing discussion describes merely exemplary embodiments illustrating the principles of the present invention, the scope of which is recited in the following claims.

## Claims

1. A balloon catheter stent delivery system (10) for treating a patient comprising:
a catheter shaft (14) extending from a catheter proximal end to a catheter distal end; the shaft defining a longitudinal axis;
a substantially inelastic balloon (12) affixed to the catheter shaft near its distal end, the balloon having a central inflatable portion between a proximal collar and a distal collar each affixed to the catheter shaft; the balloon in an initial configuration being deflated, pleated and wrapped around the catheter shaft;
a stent (34) crimped around the inflatable portion deflated balloon in the initial configuration;
a distal tip (36) defined by a portion of the catheter extending distally of a point where the distal collar of the balloon meets the catheter shaft; **characterised in that** the distal tip having a length along the longitudinal axis of 2.5 millimeters or less;
wherein the distal tip defines more than one cam surface (38, 40, 50, 52) which tapers in the distal direction;
a first of the cam surfaces defining an angle (54) of 5-20 degrees with respect to the longitudinal axis, located within 0.254 millimeters of the catheter distal end; and
a second of the cam surfaces defining an angle (56) of 18-35 degrees with respect to the longitudinal axis, located proximal of the first of the cam surfaces and within 2.0 millimeters of the catheter distal end;
such that the cam surfaces gently urge the balloon catheter to more smoothly follow the desired path during advancement to a desired site for treatment.

2. The balloon catheter stent delivery system of Claim 1, wherein the material of which the balloon is made extends to the catheter distal end.

3. The balloon catheter stent delivery system of Claim 1, wherein the first cam surface extends proximally from the catheter distal end a distance of at most 0.3 millimeters.

4. The balloon catheter stent delivery system of Claim 1, wherein an initial outer dimension of the balloon and stent are adapted for treatment of a body passage having a relevant major dimension of 2-4 millimeters.

5. The balloon catheter stent delivery system of Claim 1, further comprising a guidewire lumen extending from the catheter distal end to a proximal guidewire port opening to the exterior of the balloon catheter.

6. The balloon catheter stent delivery system of Claim 5, wherein the proximal guidewire port (30) is located between the balloon and the proximal end of the balloon catheter, in a rapid exchange configuration.

7. The balloon catheter stent delivery system of Claim 5, wherein the proximal guidewire port is located near the proximal end of the balloon catheter, in an over-the-wire configuration.

8. A method of making a balloon catheter stent delivery system, comprising the steps of:
providing a catheter shaft having a proximal and distal end and defining a longitudinal axis;
providing a substantially inelastic balloon having a central inflatable portion between a proximal collar and a distal collar;
assembling the balloon around the shaft near the distal end of the shaft;
inserting the resulting assembly within a heating die defining a tapered bore;
heating the die to a temperature greater than the melting temperature of both the balloon and shaft, while pressing the balloon and shaft into the tapered bore of the heating die, to cause a portion of the materials of the balloon and shaft to melt and seal together, simultaneously forming a distal tip defined by a portion of the catheter extending distally of a point where the distal collar of the balloon meets the catheter shaft; the distal tip having a length along the longitudinal axis of 2.5 millimeters or less;
wherein the distal tip defines more than one cam surface which tapers in the distal direction;
a first of the cam surfaces defining an angle of 5-20 degrees with respect to the longitudinal axis, located within 0.254 millimeters of the catheter distal end; and
a second of the cam surfaces defining an angle of 18-35 degrees with respect to the longitudinal axis, located proximal of the first of the cam surfaces and within 2.0 millimeters of the catheter distal end;
folding the balloon material with more than one longitudinal fold;
wrapping the folded portion around the shaft;
providing a cylindrical mesh stent; and
crimping the stent around the balloon.

## Patentansprüche

1. Ballonkatheter-Stent-Zufuhrsystem (10) zur Behandlung eines Patienten, umfassend:
einen Katheterschaft (14), der sich von einem proximalen Ende des Katheters zu einem distalen Ende des Katheters erstreckt, wobei durch den Schaft eine Längsachse definiert ist;
einen im wesentlichen unelastischen Ballon (12), der am Katheterschaft in der Nähe des distalen Endes desselben befestigt ist, wobei der Ballon einen zentralen aufblasbaren Abschnitt zwischen einer proximalen Manschette und einer distalen Manschette aufweist, die jeweils am Katheterschaft befestigt sind, wobei der Ballon in einer Anfangskonfiguration entleert, zusammengefaltet und um den Katheterschaft gewickelt ist;
einen um den aufblasbaren Abschnitt des in der Anfangskonfiguration entleerten Ballons gefalteten Stent (34);
eine durch einen sich distal von einem Punkt, an dem die distale Manschette des Ballons auf den Katheterschaft trifft, erstreckenden Abschnitt des Katheters definierte distale Spitze (36), **dadurch gekennzeichnet, daß** die distale Spitze eine Länge entlang der Längsachse von 2,5 mm oder weniger aufweist;
wobei durch die distale Spitze mehr als eine sich in der distalen Richtung verjüngende Schulteroberfläche (38, 40, 50, 52) definiert ist;
wobei durch eine erste der Schulteroberflächen, die sich innerhalb von 0,254 mm vom distalen Ende des Katheters befindet, ein Winkel (54) von 5-20 Grad im Verhältnis zur Längsachse definiert ist; und
durch eine zweite der Schulteroberflächen, die sich proximal zur ersten der Schulteroberflächen und innerhalb von 2,0 mm vom distalen Ende des Katheters befindet, ein Winkel (56) von 18-35 Grad im Verhältnis zu Längsachse definiert ist;
so daß die Schulteroberflächen den Ballonkatheter behutsam dazu zwingen, dem gewünschten Weg beim Vorschieben zu einem gewünschten Behandlungsort glatter zu folgen.

2. Ballonkatheter-Stent-Zufuhrsystem nach Anspruch 1, wobei sich das Material, aus dem der Ballon hergestellt ist, zum distalen Ende des Katheters erstreckt.

3. Ballonkatheter-Stent-Zufuhrsystem nach Anspruch 1, wobei sich die erste Schulteroberfläche proximal vom distalen Ende des Katheters über eine Distanz von höchstens 0,3 mm erstreckt.

4. Ballonkatheter-Stent-Zufuhrsystem nach Anspruch 1, wobei eine äußere Anfangsabmessung des Ballons und des Stents für eine Behandlung eines Durchgangs im Körper mit einer relevanten Hauptabmessung von 2-4 mm eingerichtet ist.

5. Ballonkatheter-Stent-Zufuhrsystem nach Anspruch 1, das des weiteren ein Führungsdrahtlumen aufweist, das sich vom distalen Ende des Katheters zu einer proximalen Führungsdrahtöffnung erstreckt, die sich zur Außenseite des Ballonkatheters öffnet.

6. Ballonkatheter-Stent-Zufuhrsystem nach Anspruch 5, wobei die proximale Öffnung (30) für den Führungsdraht zwischen dem Ballon und dem proximalen Ende des Ballonkatheters in einer Schnellaustauschkonfiguration angeordnet ist.

7. Ballonkatheter-Stent-Zufuhrsystem nach Anspruch 5, wobei sich die proximale Öffnung für den Führungsdraht in der Nähe des proximalen Endes des Ballonkatheters in einer Über-den-Draht-Konfiguration befindet.

8. Verfahren zur Herstellung eines Ballonkatheters-Stent-Zufuhrsystems, das die Schritte umfaßt:
Bereitstellen eines Katheterschafts mit einem proximalen und einem distalen Ende, durch den eine Längsachse definiert wird;
Bereitstellen eines im wesentlichen unelastischen Ballons mit einem zentralen aufblasbaren Abschnitt zwischen einer proximalen Manschette und einer distalen Manschette;
Montieren des Ballons um den Schaft in der Nähe des distalen Endes des Schafts;
Einfügen der daraus erhaltenen Anordnung in eine Heizform, durch die eine sich verjüngende Bohrung definiert wird;
Erwärmen der Form auf eine über dem Schmelzpunkt sowohl des Ballons als auch des Schafts liegende Temperatur, während der Ballon und der Schaft in die sich verjüngende Bohrung der Heizform gedrückt werden, so daß bewirkt wird, daß ein Teil der Materialien des Ballons und des Schafts schmelzen und miteinander verschweißt werden;
gleichzeitiges Formen einer distalen Spitze, die durch einen sich distal von einem Punkt, an dem die distale Manschette des Ballons auf den Katheterschaft trifft, erstreckenden Teil des Katheters definiert ist, wobei die distale Spitze eine Länge entlang der Längsachse von 2,5 mm oder weniger aufweist;
wobei durch die distale Spitze mehr als eine sich in der distalen Richtung verjüngende Schulteroberfläche definiert wird;
durch eine erste der Schulteroberflächen, die innerhalb von 0,254 mm vom distalen Ende des Katheters angeordnet ist, ein Winkel von 5-20 Grad im Verhältnis zu Längsachse definiert wird; und
durch eine zweite der Schulteroberflächen, die proximal zur ersten der Schulteroberflächen und innerhalb von 2,0 mm vom distalen Ende des Katheters angeordnet ist, ein Winkel von 18-35 Grad im Verhältnis zur Längsachse definiert wird;
Falten des Ballonmaterials mit mehr als einer Längsfaltung;
Wickeln des gefalteten Abschnitts um den Schaft;
Bereitstellen eines Stents mit einem zylindrischem Netz; und
Falten des Stents um den Ballon.

## Revendications

1. Système de pose de stent (10) par cathéter à ballonnet pour traiter un patient, comprenant :
◆ une tige (14) de cathéter s'étendant à partir d'une extrémité proximale de cathéter jusqu'à une extrémité distale de cathéter ; la tige définissant un axe longitudinal ;
◆ un ballonnet (12) sensiblement inélastique fixé sur la tige de cathéter à proximité de son extrémité distale, le ballonnet ayant une partie gonflable centrale entre un collier proximal et un collier distal fixé chacun sur la tige de cathéter ; le ballonnet dans une configuration initiale étant dégonflé, plissé et enroulé autour de la tige de cathéter ;
◆ un stent (34) serti autour de la partie gonflable du ballonnet dégonflé dans la configuration initiale ;
◆ un embout distal (36) défini par une partie du cathéter s'étendant de manière distale par rapport à un point où le collier distal du ballonnet rencontre la tige de cathéter; **caractérisé en ce que** l'embout distal a une longueur le long de l'axe longitudinal de 2,5 millimètres ou moins ;
dans lequel l'embout distal définit plus d'une surface de came (38, 40, 50, 52) qui se rétrécie progressivement dans la direction distale ;
◆ une première des surfaces de came définissant un angle (54) de 5 à 20 degrés par rapport à l'axe longitudinal, située dans les limites de 0,254 millimètres de l'extrémité distale du cathéter ; et
◆ une seconde des surfaces de came définissant un angle (56) de 18 à 35 degrés par rapport à l'axe longitudinal, positionnée de manière proximale par rapport à la première des surfaces de came et dans les limites de 2,0 millimètres de l'extrémité distale du cathéter ;
de sorte que les surfaces de came poussent progressivement le cathéter à ballonnet pour suivre plus régulièrement la trajectoire souhaitée pendant l'avancement jusqu'à un site souhaité pour le traitement.

2. Système de pose de stent par cathéter à ballonnet selon la revendication 1, dans lequel le matériau avec lequel le ballonnet est réalisé, s'étend vers l'extrémité distale du cathéter.

3. Système de pose de stent par cathéter à ballonnet selon la revendication 1, dans lequel la première surface de came s'étend de manière proximale à partir de l'extrémité distale du cathéter, sur une distance au maximum de 0,3 millimètres.

4. Système de pose de stent par cathéter à ballonnet selon la revendication 1, dans lequel les dimensions externes initiales du ballonnet et du stent sont adaptées pour le traitement d'un passage corporel ayant une dimension principale appropriée de 2 à 4 millimètres.

5. Système de pose de stent par cathéter à ballonnet selon la revendication 1, comprenant en outre une lumière de fil guide s'étendant de l'extrémité distale du cathéter jusqu'à un orifice de fil guide proximal s'ouvrant sur l'extérieur du cathéter à ballonnet.

6. Système de pose de stent par cathéter à ballonnet selon la revendication 5, dans lequel l'orifice de fil guide proximal (30) est situé entre le ballonnet et l'extrémité proximale du cathéter à ballonnet, selon une configuration d'échange rapide.

7. Système de pose de stent par cathéter à ballonnet selon la revendication 5, dans lequel l'orifice de fil guide proximal est situé à proximité de l'extrémité proximale du cathéter à ballonnet, dans une configuration sur le fil.

8. Procédé pour fabriquer un système de pose de stent par cathéter à ballonnet, comprenant les étapes consistant à :
◆ prévoir une tige de cathéter ayant une extrémité proximale et une extrémité distale et définissant un axe longitudinal ;
◆ prévoir un ballonnet sensiblement inélastique ayant une partie gonflable centrale entre un collier proximal et un collier distal ;
◆ assembler le ballonnet autour de la tige à proximité de l'extrémité distale de la tige ;
◆ insérer l'ensemble résultant dans une filière chauffante définissant un alésage progressivement rétréci ;
◆ chauffer la filière à une température supérieure à la température de fusion à la fois du ballonnet et de la tige, tout en comprimant le ballonnet et la tige dans l'alésage progressivement rétréci de la filière chauffante, pour amener une partie des matériaux du ballonnet et de la tige à fondre et se souder ensemble, formant simultanément un embout distal défini par une partie du cathéter s'étendant de manière distale par rapport à un point où le collier distal du ballonnet rencontre la tige de cathéter ; l'embout distal ayant une longueur le long de l'axe longitudinal de 2,5 millimètres ou moins ;
dans lequel l'embout distal définit plus d'une surface de came qui se rétrécit progressivement dans la direction distale ;
une première des surfaces de came définissant un angle de 5 à 20 degrés par rapport à l'axe longitudinal, située dans les limites de 0,254 millimètres de l'extrémité distale du cathéter ; et
une seconde des surfaces de came définissant un angle de 18 à 35 degrés par rapport à l'axe longitudinal, située de manière proximale par rapport à la première des surfaces de came et dans les limites de 2,0 millimètres de l'extrémité distale de cathéter ;
◆ plier le matériau de ballonnet avec plus d'un pli longitudinal ;
◆ enrouler la partie pliée autour de la tige ;
◆ prévoir un stent en maille cylindrique ; et
◆ sertir le stent autour du ballonnet.
